(19) Europäisches Patentamt
European Patent Office
Office européen des brevets

(11) Publication number: **0 172 007 B1**

(12) **EUROPEAN PATENT SPECIFICATION**

(45) Date of publication of patent specification: **22.05.91**

(51) Int. Cl.$^5$: **A61K 9/127**, A61K 37/66

(21) Application number: **85305672.9**

(22) Date of filing: **09.08.85**

(54) **Stable liposomes with aqueous-soluble medicaments and methods for their preparation.**

(30) Priority: **10.08.84 US 639638**
**31.05.85 US 739729**

(43) Date of publication of application:
**19.02.86 Bulletin 86/08**

(45) Publication of the grant of the patent:
**22.05.91 Bulletin 91/21**

(84) Designated Contracting States:
**AT BE CH DE FR GB IT LI LU NL SE**

(56) References cited:
**GB-A- 1 575 343**
**US-A- 4 186 183**

**CHEMICAL ABSTRACTS, vol. 97, no. 10, September 1982, page 414, abstract no. 78825v, Columbus, Ohio, US; D. GURARI-ROTMAN et al.: "Encapsulation of human fibroblast interferon activity in liposomes", & BIOCHEM. BIOPHYS. RES. COMMUN. 1982, 107(1), 136-43**

(73) Proprietor: **SYNTEX (U.S.A.) INC.**
**3401 Hillview Avenue**
**Palo Alto California 94303(US)**

(72) Inventor: **Felgner, Philip L.**
**1080 Fremont Avenue**
**Los Altos California 94022(US)**
Inventor: **Eppstein, Deborah A.**
**2290 Louis Road**
**Palo Alto California 94303(US)**

(74) Representative: **Armitage, Ian Michael et al**
**MEWBURN ELLIS & CO. 2/3 Cursitor Street**
**London EC4A 1BQ(GB)**

**EP 0 172 007 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 100, no. 18, April 1984, page 358, abstract no. 144909j, Columbus, Ohio, US; H. YOSHIKAWA et al.: "A method to potentiate enteral absorption of interferon and selective delivery into lymphatics", & J. PHARMACOBIO-DYN. 1984, 7(1), 59-62

CHEMICAL ABSTRACTS, vol. 94, no. 16, April 1981, page 381, abstract no. 127289x, Columbus, Ohio, US; M. OBLADEN et al.: "Multilamellar liposome preparation and surface activity of synthetic phospholipids", & PROG. RESPIR, RES. 1981, 15(Clin. Importance Surfactant Defects), 177-87

CHEMICAL ABSTRACTS, vol. 92, no. 20, May 1980, page 341, abstract no. 169159u, Columbus, Ohio, US; E. MAYHEW et al.: "Role of cholesterol in enhancing the antitumor activity of cytosine arabinoside entrapped in liposomes", CANCER TREAT. REP. 1979, 63(11-12) 1923-8

## Description

### Field of the Invention

This invention relates to a new, stable liposome composition containing an aqueous-soluble medicament.

This invention also relates to a method for preparing liposomes for use with aqueous-soluble medicaments, specifically a method that employs lyophilization of the lipid prior to hydration with a medicament-containing solution.

### Related Disclosures

The standard method for preparing liposomes is to first dissolve the lipid in an organic solvent such as methanol, ethanol, dichloromethane or the like, and then dry down the solution using either rotary evaporation or a gas stream, e.g., nitrogen. The result is a thin lipid film distributed on the sides of the flask. An aqueous solution, usually a buffer solution if multilamellar liposomes are desired, is then added to the dry lipid film in order to suspend the lipid film and effect liposome formation.

There are three stages in the formation of the liposomes from the dried film; (i) swelling, (ii) vesicle formation, and (iii) dispersion of the liposomes. In the swelling stage, water can be observed to give rise to a visible increase in the thickness of the dried film and the film becomes more opaque. Water readily penetrates the film at this stage, but many solutes tend to be excluded. After swelling, the hydrated lipid film breaks up into discreet vesicles which then disperse, see e.g. US 4186183. Since lipid vesicles are permeable to water but exclude many solutes, the capture efficiency of this technique is often low. It is one object of this invention to provide a technique that will give a greater solute capture efficiency.

An additional disadvantage of the standard technique is that liposomes formed from dried films frequently have defects which lead to liposome instability, leakage of encapsulated solutes and oxidation of the lipid material. These defects arise because lipids oriented in the film must be reoriented to form the liposomes. The standard technique does not provide for the smooth reorganization of the lipids into the highly ordered state which exists in ideal liposomes. The technique herein provides for more advantageous lipid rearrangement, as the lyophilized lipid is initially highly disorganized and is organized first in the presence of the aqueous solution. The absence of a pre-existing organized film prevents exclusion of solutes as the liposomes form.

Additionally, the standard technique offen requires vigorous shaking, frequently sonication, to form and disperse the liposomes. Unless performed under an inert atmosphere, this step will increase the amount of oxygen dissolved in the suspension, with a concomitant increase in the oxidation of the liposomes and active compounds in the suspension. Vigorous shaking or sonication may also partially or completely denature or inactivate sensitive compounds, including proteins such as interferon and shear strands of polynucleotides. The method of the invention avoids the necessity for any violent treatment: the liposomes are formed and dispersed almost instantaneously upon addition of the hydrating solution. The method of the invention does not increase the amount of dissolved air in the suspension, and so may be practiced without the need for an inert atmosphere.

This procedure also facilitates the preparation of more concentrated suspensions of liposomes, and it is this more concentrated preparation that in part leads to the improved capture efficiency of aqueous-soluble compounds. More concentrated preparations lead to improved capture because the internal volume of the liposomes is a greater fraction of the total volume of the suspension when the concentration of liposomes in the preparation is high. This improved concentration, coupled with reduced solute exclusion, results in a capture efficiency much higher than that realizable by prior art methods. After formation of the concentrated liposome suspension, the suspension may be diluted to any lesser concentration, if so desired.

The instant invention involves selecting a lipid satisfactory for preparing liposomes, an organic solvent that can be frozen and lyophilized, and an aqueous-soluble medicament. The appropriate lipid is dissolved in the solvent and the resulting solution frozen and placed under reduced pressure until dry, with a cold trap to recover the evaporating solvent. The lyophilized lipid can then be suspended in an appropriate aqueous vehicle in which the aqueous-soluble medicament has been dissolved. The resulting suspension contains liposomes encapsulating an aqueous-soluble medicament, which liposomes have increased structural integrity, greater stability and less leakage as compared with liposomes prepared by the method of drying down the lipid solution using a gas stream or rotary evaporation. Because the medicament is added in the aqueous solution, rather than being suspended in the organic solvent with the lipids, this method has utility

for proteins and other materials that are inactivated or degraded by contact with organic solvents.

Besides the unique structural and chemical stability properties imparted by this method, this method usually increases the efficacy of the encapsulated medicament.

A method for preparing liposomes with lipid-soluble or lipid-bound medicaments using lyophilization is disclosed in U.S. Pat. Nos. 4,311,712 and 4,370,349 to Evans, et al. However, Evans, et al. teach that their methods are not suitable for aqueous-soluble medicaments (such as interferon) that are not otherwise lipid-bound, contrary to the utility of the present invention. Evans' first method requires the practitioner to combine the lipids and lipid-soluble medicament in an organic solvent, lyophilize the mixture, and hydrate the lyophilized powder with an aqueous solution to form liposomes. Evans' second method involves lyophilizing an aqueous suspension of preformed liposomes containing medicaments, where the liposomes have been prepared by, e.g., the dried film technique (see also GB 1575343 and Biochem. and Biophys. Res. Com. Vol 107, No 1, 1982).

A method for improving storage stability by lyophilizing aqueous suspensions of liposomes is disclosed by Vanlerberghe in U.S. Pat. No. 4,247,411. However, Vanlerberghe's method requires that the liposomes first be prepared by some method other than lyophilization, e.g., by hydration of dried lipid films. Schneider teaches a similar method in U.S. Pat. No. 4,229,360, which includes an aqueous-soluble additive to prevent the lyophilized lipids from forming a tarry residue. However, the practice of the instant invention produces a powdery lyophilized lipid material without the need for additives.

Additionally, it has surprisingly been found that the method of the invention may be used to encapsulate interferon, without loss of activity, with a capture efficiency of nearly 100%.

Another surprising result of the invention is the gradual, controlled release of active interferon near the site of administration. When administered topically, subcutaneously or intramuscularly, liposome-encapsulated interferon remains near the site of injection or local administration and is released gradually over an extended period, e.g., about 1 to 14 days. The release characteristics are a function of the liposome composition. These characteristics make such a formulation ideal for treating local lesions, tumors, and other injuries, e.g., herpes virus lesions and genital warts.

## SUMMARY OF THE INVENTION

This invention relates to liposomes containing aqueous-soluble medicaments, which are more stable, have a higher capture efficiency, and are more easily prepared than known liposomes.

This invention also relates to a process for preparing liposomes with aqueous-soluble medicaments, especially interferon, which process comprises dissolving an appropriate lipid in a lyophilizable organic solvent, removing said solvent by lyophilization, and hydrating the lyophilized lipid with an aqueous solution containing an aqueous-soluble medicament.

This invention especially relates to liposomes containing active interferon, which are highly stable, have a high capture efficiency, are easily prepared, and which, when administered, usually by topical application or by subcutaneous or intramuscular injection, remain near the site of administration, and release interferon over a period of 1 to 14 days.

## DEFINITIONS

As used herein, the term "suitable aqueous-soluble medicament" refers to a pharmaceutical agent which is useful for treating a disease or disorder in a mammal, and which is soluble in water or aqueous solutions. Preferred medicaments are biologically active proteins and polypeptides which are hydrophilic.

The term "treatment" as used herein covers any treatment of a disease or disorder in a mammal, and includes:

(i) preventing the disease from occurring in a subject that is not diagnosed as having the disease;
(ii) inhibiting the disease, i.e., arresting its development; or
(iii) relieving the disease, i.e., causing regression of the disease.

The term "mammal" as used herein includes humans and domestic mammals such as cattle, horses, swine, sheep, dogs, cats, goats and the like, as well as wild animals.

A pharmaceutically acceptable anion is some anion which itself is not toxic or otherwise not pharmaceutically unacceptable and which does not render the compound pharmaceutically unacceptable. Examples of such anions are the halide anions fluoride, chloride, bromide, and iodide. Inorganic anions such as sulphate, sulfite, phosphate, and nitrate may also be used. Organic anions may be derived from simple organic acids

such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methane sulfonic acid, ethane sulfonic acid, p-toluenesulfonic acid, and the like.

As used in this Specification and the appended Claims, the term "liposome" is intended to cover any concentric lipid bilayer structure consisting of closed concentric lamellae enclosing one or more aqueous-containing compartments and prepared from a pharmaceutically acceptable lipid. Liposomes are microscopic vesicles, generally spherically shaped, formed from one or more concentric layers (lamellae) of lipid molecules having a lipophilic and hydrophilic moiety. Most frequently, liposomes are composed of water insoluble amphipathic phospholipids which form bilayer structures spontaneously in aqueous solution. Regardless of the overall shape, liposome bilayers are organized as closed concentric lamellae with an aqueous layer separating each lamella from its neighbor. Liposomes contain at least one lipid bilayer, the molecules of said layer being so oriented that the hydrophilic end of each molecule is in contact with the aqueous phase. The molecules comprising the liposomal membrane can be schematically represented by the structure X-Y, where X represents the hydrophobic portion of the molecule and Y the hydrophilic portion. In aqueous solution the molecules arrange themselves into concentric lamellae separated by an aqueous layer which can be represented schematically as YX/XY-W-YX/XY, where the hydrophobic portions of the molecules form the inner portion of each layer, and each two layer is separated from the next by an aqueous layer, here represented by -W-.

Liposomes are most frequently prepared from phospholipids, but other molecules of similar molecular shape and dimensions having both a lipophilic and a hydrophilic moiety can be used to prepare liposomes. For the purposes of this Specification, all such appropriate, liposome-forming molecules will be referred to herein as "lipids." Molecules of other shapes and dimensions may be added, up to an experimentally-determinable mole fraction, without destroying the liposomes' lamellar structure.

Liposome vesicle size is highly variable and depends on the method of manufacture thereof. Generally, liposomes have a 20 nm to 30,000 nm diameter, with aqueous intralamellar layers of 3-10 nm.

Liposomes can be classified into three categories based on their overall size and the nature of the lamellar structure. The three classifications are small unilamellar vesicles (SUV), multilamellar vesicles (MLV), and large unilamellar vesicles (LUV), using the nomenclature developed at the New York Academy of Sciences meeting on "Liposomes and Their Use in Biology and Medicine" of September 1977. In addition, MLVs prepared using the lyophilization methods of the invention are herein designated LMLV.

SUVs range in diameter from approximately 20 to 50 nm and consist of a single lipid bilayer surrounding an aqueous compartment. A characteristic of SUVs is that a large amount of the total lipid, about 70%, is located in the outer layer of the bilayer. In addition, the small radius of curvature imposes packing strain in the lipid layers resulting in them being rendered metastable in certain circumstances.

The most frequently encountered and easily prepared liposomes are multilamellar vesicles (MLV). Where SUVs are single compartmental vesicles of fairly uniform size, MLVs vary greatly in diameter up to about 30,000 nm and are multi-compartmental in their structure. Large unilamellar vesicles (LUV) are so-named because of their large diameter, which ranges from about 600 nm up to 30 microns.

Liposomes may also be prepared incorporating recognition macromolecules to bind liposomes selectively to particular cells. Such recognition macromolecules may be, for example, antibodies, plant lectins, desialylated glycoproteins, and the like. Such materials have been attached to liposomes by covalent or non-covalent forces.

Liposomes may be prepared from any compound having the requisite hydrophilic and hydrophobic activity. Many lipids have been developed for making liposomes wherein the hydrophilic group is phosphate, carboxylate, sulfate, amino, hydroxyl or a choline group, and the lipophilic group is alkyl, alkenyl, polyoxyalkenyl or alkyl substituted with aromatic or cycloalkyl, for example, ternary and complex lipids, glycerides, cerides, etholides and sterides.

Liposomes may be anionic, cationic or neutral depending upon the choice of hydrophilic group. For instance, when a compound with a phosphate or a sulfate group is used, the resulting liposomes will be anionic. When amino-containing lipids are used, the liposomes will have a positive charge, and will be cationic liposomes. When polyethylenoxy or glycol groups are present in the lipid, neutral liposomes are obtained. Additional compounds suitable for forming liposomes may be found in McCutchen's Detergents and Emulsifiers and McCutchen's Functional Materials, Allured Publishing Company, Richwood, N.J., U.S.A.

Preferred lipids are phospholipid-related materials, such as lecithin, phosphatidylethanolamine, lysolethicin, lysophosphatidylethanolamine, phosphatidylserine, phosphatidylinositol, sphingomyelin, cephalin, cardiolipin, phosphatidic acid, cerebrosides, dicetylphosphate, phosphatidylcholine and dipalmitoylphosphatidylglycerol. Additional non-phosphorous containing lipids are, e.g., stearylamine, dodecylamine, hexadecylamine, acetyl palmitate, glycerol ricinoleate, hexadecyl stereate, isopropyl

myristate, amphoteric acrylic polymers, triethanolamine-lauryl sulfate, alkyl-aryl sulfate polyethyloxylated fatty acid amides, and the like. Particularly preferred non-phosphorous containing lipids have the formula I:

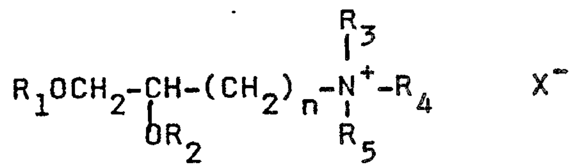

(I)

wherein $R_1$ and $R_2$ are independently alkyl or alkenyl of 6-22 carbons; $R_3$, $R_4$ and $R_5$ are each independently hydro, alkyl of 1-8 carbons, aryl or arylalkyl of 7-11 carbons; n is an integer from 1 to 8; and X is a pharmaceutically acceptable anion.

Compounds of formula I are made by two procedures, depending on the value of n. Where n is 1, the compounds of formula I may be derived from D-mannitol. The two central hydroxy groups of mannitol are first protected by formation of a ketal, for example by formation of the acetonide. The four remaining hydroxy groups are then converted to ethers using the appropriate long chain fatty acid. This intermediate is then cleaved into two 3-carbon aldehydes, wherein two carbons are substituted with a long chain alkoxy or alkenoxy group. The aldehyde functionality is then converted to a tertiary amine, and then further converted to either the acid addition salt or a quaternary ammonium compound. This process is exemplified by Reaction Scheme I as follows:

REACTION SCHEME I

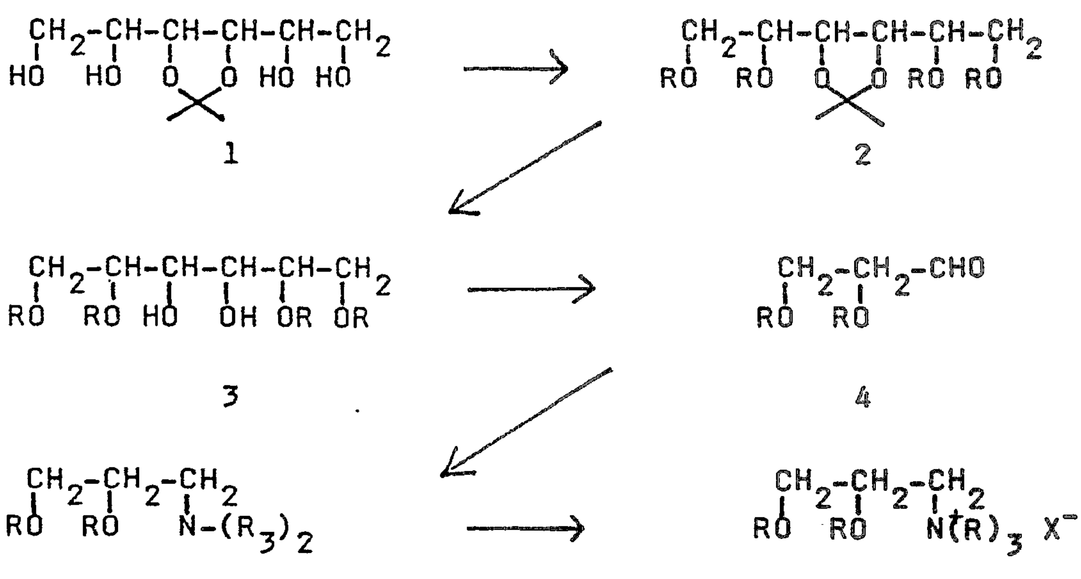

1 2 3 4 5 I

The D-mannitol-3,4-acetonide of formula I is known and may be prepared by the methods of Willing, L. F., J. Chem. Soc., 13 (1946); or Morpain, C. & Tisserand, M., J. Chem. Soc., 1379 (1979).

To prepare compound 2, the acetonide is dissolved in an appropriate polar solvent such as dimethylformamide, diethylformamide, or the like. To this is added a strong base, such as sodium hydride, at room temperature. This mixture is then heated, preferably between about 30°-100° C, more preferably to about 50° C, with stirring for approximately 30-90 minutes, preferably about 60 minutes. An alkylating agent of the desired chain length is then added, exemplified by the toluenesulfonate ester of oleyl alcohol or by 1-bromohexadecane. Following addition of the alkylating agent, the temperature is increased to about 50°-150° C, preferably about 90° C, with additional stirring over a period of up to 2 hours, preferably about 1

hour. The base is added in a molar amount equal to the molar amount of acetonide being used, followed by addition of an equimolar amount of the alkylating agent selected. This sequence of adding a molar amount of base with heating followed by a molar amount of the alkylating agent with heating and stirring is repeated four times (for a total of five times) in order to effect the formation of compound 2.

Compound 3 is made by hydrolyzing the ketal, illustrated by the acetonide. The hydrolysis is carried out as a single phase reaction using a polar, water-soluble organic solvent such as tetrahydrofuran. Preferably, the hydrolysis will be effected using a 10% solution of water in trifluoroacetic acid. The solution of the mannitol tetraether in organic solvent and aqueous acid solution is stirred for approximately one hour at a slightly elevated temperature, preferably about 50 °C. The solvent is then evaporated and residual acid removed by azeotropic distillation using a solvent such as toluene.

The aldehyde 4 is made by treating diol 3 with an oxidant, preferably one, such as lead tetraacetate, in a suitable solvent, e.g., chloroform. A slight molar excess of lead tetraacetate is used to effect the reaction. The mixture is stirred at about ambient temperature for up to 4 hours, preferably about 2 hours, at which time the excess lead tetraacetate is quenched by addition of ethylene glycol followed quickly by the addition of a substantial amount of water. The resulting crude aldehyde is recovered by conventional means and may be used without further purification directly in the next step.

To effect the formation of the compound 5 amine, a dialkylamine hydrochloride is dissolved in an alcohol, preferably methanol, to which solution is added a two-thirds molar amount of anhydrous sodium acetate. This mixture is stirred for about an hour at ambient temperature and the resulting sodium chloride is filtered off. The methanol solution is then added to the crude aldehyde prepared above. A second solvent, preferably tetrahydrofuran, is then added to this mixture followed by molecular sieves. To this mixture is then added a reducing agent, preferably sodium cyanoborohydride, in a slight molar excess, and the mixture stirred at a slightly elevated temperature, preferably about 40-60 °C, for up to 3 days. This product is then converted to the hydrochloride salt by addition of an organic solvent through which HCl gas has been bubbled.

The quaternary ammonium compound is then prepared by condensing an alkyl chloride into a reaction vessel containing the dialkylamine material, after which the reaction vessel is sealed and heated to about 50 °-100 °C, preferably about 70 °C, for up to 48 hours. This procedure affords the trialkylammonium chloride product of formula 6 (formula I).

Alternatively, compounds of Formula I where n is 1 and $R_1$ and $R_2$ are not the same can be prepared by the flowchart of Reaction Scheme II:

REACTION SCHEME II

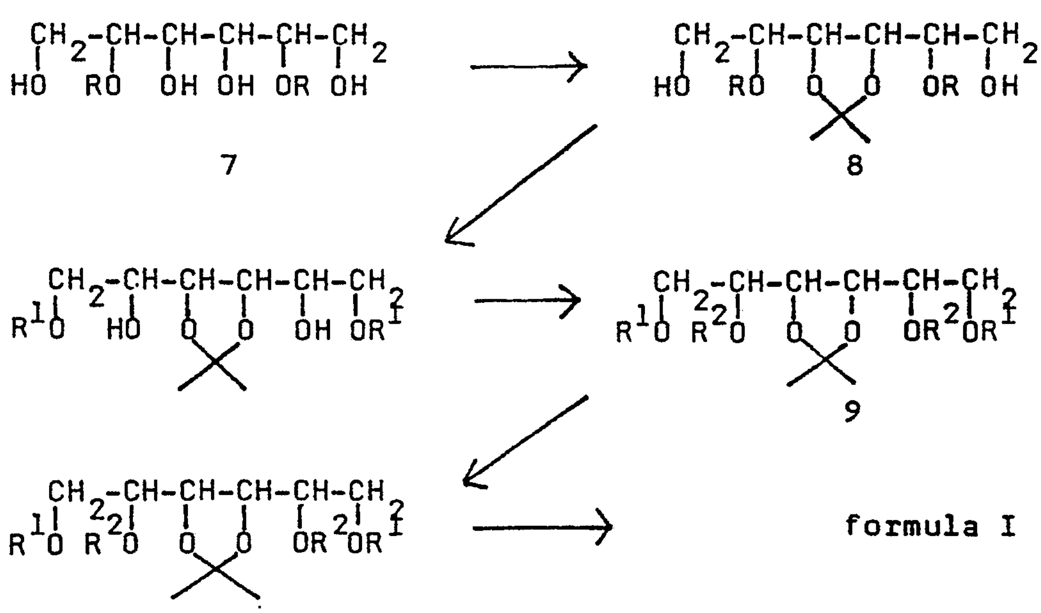
7
8
9
formula I

In this reacton scheme, R is benzyl, and $R^1$ and $R^2$ are defined herein above, $R^1$ being different from

$R^2$.

The 2,5-dibenzyl-D-mannitol of formula 7 was prepared by the procedure of Baggett, N., & Stribblehil, J. Chem. Soc. Perkin I, 1323 (1977). The dibenzyl-D-mannitol is dissolved in a dry ketal-forming solvent, for example, acetone, to which is added a half molar amount of copper sulphate and a small amount of concentrated sulphuric acid. This solution is stirred at ambient temperature for about 48 hours, at which time the mixture is quenched by means of a weak base, preferably sodium carbonate, and then stirred for an additional period of time to effect the reaction. The solvent is also the source of the ketal.

The position 1 and position 6 hydroxyl groups are then etherified by means of a strong base and an 1-haloalkyl, or 1-haloalkenyl moiety. The ketal is dissolved in a non-polar solvent, such as xylene, toluene, or the like, to which is added a powdered base, such as powdered potassium hydroxide and the 1-haloalkyl or 1-haloalkenyl material. This mixture is heated at reflux for about 4 hours in order to effect formation of compound 8.

The two benzyl groups are then removed by catalytic hydrogenolysis in an appropriate solvent such as tetrahydrofuran/methanol using a heavy metal catalyst such as 10% palladium on carbon is used. The reaction is carried out in an appropriate hydrogenolysis device, in this instance with heating to about 60°-80° C, for about 48 hours under about 60 psi of hydrogen.

The diol obtained from the preceding hydrogenolysis is etherified in the same manner described above for preparing compound 9.

Once the tetrasubstituted D-mannitol-3,4-ketal is obtained, it is converted to a compound of formula I by the series of steps recited above for conversion of formula 2 to formula 6.

Those compounds wherein n is 2-8 are prepared from the corresponding triol. The schematic for this reaction sequence is set forth in Reaction Scheme III which follows. This scheme may also be used for preparing compounds where n is 1.

REACTION SCHEME III

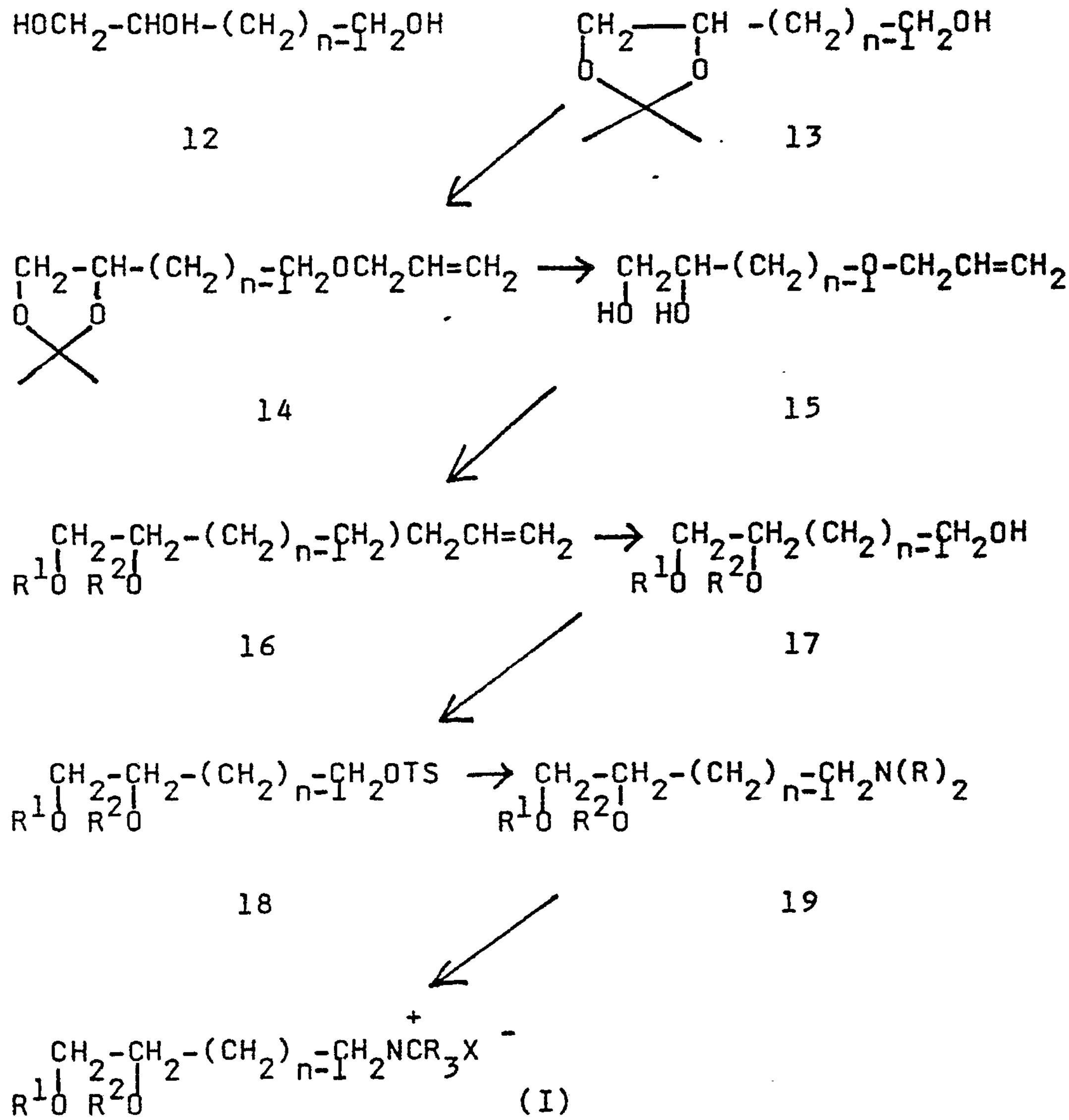

12 13 14 15 16 17 18 19 (I)

In this reaction scheme, $R^1$ and $R^2$ are the same as defined herein above. In formula I, R is the same as $R^3$, $R^4$ and $R^5$.

The compounds of formula 12 are known in the literature and may be purchased from a chemical supply house, or may be prepared by on of ordinary skill in the art.

The ketal of formula 13, preferably the acetonide, is prepared by dissolving the appropriate triol in acetone with the addition of a small amount of concentrated sulphuric acid. This reaction may be effected by stirring the solution for up to about 4 hours at room temperature, preferably about 2 hours. The resulting ketal is recovered by standard separatory means.

The unreacted position-l hydroxyl group is then protected by forming an allyl ether. This reaction is carried out by dissolving the alcohol in a dry dipolar aprotic solvent, such as dimethylformamide. A strong base, such as sodium hydride (an equal molar amount), is added to the alcohol which is stirred at ambient temperature and then warmed to between 80°-100°C for an equal period. Allyl chloride in about a 50% molar excess is then added at the elevated temperature with stirring. Stirring and heating is continued for another 30 to 120 minutes, preferably about 60 minutes. The product is then extracted and further purified by chromatography.

The ketal is then hydrolyzed by means of a dilute solution of a strong acid, for example, $1\overline{N}$ HCl in a polar solvent, such as methanol, ethanol, or the like. The reaction mixture is heated to effect the hydrolysis. Preferably, the solution is heated to about 50°C for about 2 hours.

The diol is converted to the diether in the same manner as described above for conversion of formula 1 to formula 2. Here again the etherification carried out in a dry dipolar aprotic solvent, such as dimethylformamide, using a strong base, such as sodium hydride, and the tosyl ester or halide of the appropriate alkyl

or alkenyl alcohol. The reaction is repeated twice using a one molar equivalent of alkylating agent each time. As described previously, the reaction is effected at an elevated temperature, preferably between 50° - 150° C, more specifically at about 90° C.

The position-l allyl ether is then hydrolyzed by means of Wilkinson's catalyst [tris(triphenylphosphine)-rhodium chloride] in an acidic medium. The solvent should be a polar solvent such as ethanol, preferably with a co-solvent such as tetrahydrofuran. The triether/catalyst mixture is refluxed for several hours, preferably about 3 hours, at which time additional acid (IN HCl) is added and refluxing continued for several more hours, approximately 3-4. These conditions effect hydrolysis of the allyl ether.

The alcohol is then converted to an amine by first creating an intermediate p-toluenesulfonate ester to which is added a dialkylamine to effect formation of the amine compound. By way of illustration, the alcohol is dissolved in a suitable solvent, such as pyridine, to which is added p-toluenesulfonyl chloride. This mixture is stirred overnight at ambient temperature, then cooled in ice water and the product recovered by extraction means. The crude product is immediately dissolved in a dialkylamine, preferably dimethylamine, and placed in a sealed container at ambient temperature for about 1 day to effect formation of the dialkylamine.

The dialkylamine is most conveniently converted to an acid addition salt, preferably a hydrochloride salt, as a means of isolating the product.

The quaternary ammonium product is then prepared in the same manner as described hereinabove for the preparation of formula 6.

The extent of the controlled release of the invention depends on the lipid composition of the liposomes. Lipids conferring controlled release characteristics generally have a saturated hydrocarbon "tail" of 14 to 24 carbon atoms. The release rate may be increased by using lipids having unsaturated hydrocarbon tails in addition to or instead of lipids with saturated hydrocarbon tails. A preferred mixture of lipids is a 7:3 mixture of dioleoylphosphatidylcholine and dioleoylphosphatidylglycerol.

Various additives can be combined with the lipid so as to modify the liposomes' permeability characteristics or the superficial charge on the spheres. Representative additives include long chain alcohols and diols, sterols such as cholesterol, long chain amines and their quaternary ammonium derivatives, dihydroxy alkylamines, polyoxyethylated fatty amines, esters of long acid amino alcohols, their salts and quaternary ammonium derivatives, phosphoric esters of fatty alcohols such as sodium dicetyl phosphate, alkyl sulfates such as sodium acetyl sulfate, certain polymers such as polypeptides, and proteins.

It should be understood that the lipids and/or additives used to prepare liposomes may be used in any combination or obtained from any natural or synthetic source.

The solvents which may be used in this process are freezable organic solvents which dissolve the chosen lipid(s) and which are capable of undergoing sublimation under reasonable industrial or research conditions. A freezable solvent appropriate to the practice of the invention is one which is capable of being removed by lyophilization, as that term is commonly used in the art. Examples of such solvents are alcohols such as propanol, isopropanol, butanol, tert-butanol, iso-butanol, pentanol, and hexanol. Alternatively, cyclohexane, cyclopentane, benzene, toluene, and the like may be used. Mixtures of two or more of these solvents may be used in the practice of this invention.

The solvent is removed in this method by lyophilization, also known as freeze drying. This well-known process employs the physical phenomena of sublimation whereby a solid becomes a gas without passing through a liquid phase. In general, a composition comprising a non-subliming material and a second material capable of undergoing sublimation is frozen. A vacuum is then applied downstream from a condensation unit, causing the sublimable material to evaporate and freeze in the condensor. This process is continued until all materials capable of undergoing sublimation at that pressure are removed from the composition.

While this process may be applied to a wide variety of solutions and solids which contain sublimable materials, for the purposes of this invention the process is concerned primarily with solvent/solute systems. For example, a solute (a lipid with or without an active ingredient) is dissolved in an appropriate solvent and the solution frozen in an appropriate container. The container is then attached to a vacuum source which has a condensor between the sample and the vacuum source. The condensor is activated and the system evacuated while keeping the sample frozen, resulting in a dry residue of the solute.

The term "capture efficiency" as used herein refers to the initial percentage of aqueous-soluble medicament that is encapsulated in liposomes. For example, if a lyophilized lipid powder is hydrated with a solution containing 10 mg of medicament, and 9 mg of medicament is encapsulated on formation of the liposomes, the liposome composition would have a capture efficiency of 90%. Dried film techniques frequently result in low capture efficiencies with aqueous-soluble medicaments, theoretically because an

organized film is able to exclude solutes from the solution hydrating the liposomal interiors.

Mammalian interferons are commonly classified into three basic, distinguishable types, denoted α-, β-, and γ-interferon. Alpha interferon (αIFN) is usually derived from mammalian leukocyte cells. Beta interferon (βIFN) is usually derived from mammalian fibroblast cells. Gamma interferon (γIFN) is usually derived from mammalian T lymphocyte cells. As used herein, "β-interferon" includes βIFN derived both from natural sources, including without limitation human, bovine, and equine, and derived by recombinant DNA techniques. It also includes modified forms of βIFN; e.g., by glycosylation, methylation, or substitution and/or deletion of a limited number of amino acids. As used herein, HuβIFN refers to human βIFN, and rHuβIFN refers to HuβIFN produced using recombinant DNA techniques. MuβIFN refers to murine (mouse) βIFN. The term "rHuβ$IFN_{ser17}$" refers to rHuβIFN in which the seventeenth amino acid has been replaced with serine. LMLV-βIFN refers to βIFN encapsulated in lyophilized multilamellar vesicles, as prepared by the method of the invention.

Interferon concentrations are commonly expressed as standard "units" (U) which are internationally accepted and documented, and relate to the potency of a given quantity of interferon to inhibit virus replication under standard conditions.

βIFN may be collected from natural sources by methods taught in the art (for example, by the methods taught in U.S. Pat. No. 4,007,086, incorporated herein by reference), or may be obtained through recombinant DNA techniques. Alternatively, α-, β-, and γ-IFN may be obtained from commercial sources. βIFN may be purified, e.g., by the process taught by U.S. Pat. No. 4,450,103, which is incorporated herein by reference, and by other methods taught in the art. See e.g., Tan, Y.H. et al, J. Biol. Chem., 254, 8067-73 (1979), Knight, Jr. E., et al, Science, 207, 525-26 (1979), Okamura, H. et al, Biochemistry, 19, 3831-35 (1980). The rHuβ$IFN_{ser17}$ used in the examples was obtained from Cetus Corp. and Triton Biosciences Inc., but may be made by the following method:

rHuβ$IFN_{ser17}$ is produced by modifying DNA sequences which code for βIFN, then manipulating microorganisms to express the modified DNA as protein. When the first base of codon 17 (deoxythymidine) of the sense strand of the DNA sequence which codes for the mature βIFN is replaced with adenine, the cysteine residue at position 17 in the amino acid sequence of βIFN is replaced by serine. By changing T to other bases, and by changing other bases in codon 17, cysteine may be replaced with other amino acids. The site-specific mutagenesis is induced using a synthetic 17-nucleotide primer having the sequence GCAATTTTCAGAGTCAG which is identical to a seventeen nucleotide sequence on the sense strand of the βIFN gene in the region of codon 17 except for a single base mismatch at the first base of codon 17. (As used herein, C = deoxycytidine, T = deoxythymidine, A = deoxyadenosine, and G = deoxyguanosine.) The mismatch is at nucleotide 12 in the primer. The 17-mer is hybridized to single-stranded M13 phage DNA which carries the antisense strand of the βIFN gene. The oligonucleotide primer is then extended on the DNA using DNA polymerase I Klenow fragment (a fragment of DNA polymerase I lacking the 5'-exonuclease subunit) and the resulting double-strand DNA (dsDNA) is converted to closed circular DNA with $T_4$ ligase. Replication of the resulting mutational heteroduplex yields clones from the DNA strand containing the mismatch. Mutant clones may be identified and screened by the appearance or disappearance of specific restriction sites, antibiotic resistance or sensitivity, or by other methods known in the art. When cysteine is substituted by serine, the substitution of T by A results in the creation of a new Hinf I restriction site in the structural gene. (A restriction site is a point in a DNA sequence that is recognized and cleaved by a particular restriction enzyme. A Hinf I restriction site is a restriction site recognized by Hinf I endonuclease.) The mutant clone is identified by using the oligonucleotide primer as a probe in a hybridization screening of the mutated phage plaques. The primer will have a single mismatch when hybridized to the parent but will have a perfect match when hybridized to the mutated phage DNA. Hybridization conditions can then be devised where the oligonucleotide primer will preferentially hybridize to the mutated DNA but not to the parent DNA. The newly generated Hinf I site also serves as a means of confirming the single base mutation in the βIFN gene.

The M13 phage DNA carrying the mutated gene is isolated and spliced into an appropriate expression vector, such as plasmid pTrp3, and E. coli strain MM294 is transformed with the vector. Suitable growth media for culturing the transformants and their progeny are known to those skilled in the art. The expressed mutein (protein derived from a mutated gene) of βIFN is isolated, purified and characterized.

As practiced herein, a liposome-forming lipid is dissolved in an appropriate solvent. The solution is transferred to an appropriate container, if not already in such, and frozen by any convenient and appropriate means. For example, liquid nitrogen, dry ice/acetone or a refrigeration system may be used to freeze the material. Once frozen, the container is attached to a vacuum system which has an intermediate condensor and the pressure reduced as low as possible within the constraints of the system, usually to about 0.1 to 1 mmHg. This vacuum is maintained until the solvent has been removed. It may be necessary to cool the

frozen solution during the sublimation process in order to prevent the sample liquifying. Once all solvent has been pumped from the frozen sample, the vacuum is broken and the dried material recovered. Depending on the lipids selected, it may he necessary to maintain the lyophilized powder at low temperature to prevent the powder from collapsing into an oil.

Liposomes are formed from the dry lipid by adding an aqueous solution containing medicament (e.g., βIFN) to hydrate the lipid layer: The liposomes are dispersed by gently shaking or swirling the container.

## DETAILED DESCRIPTION AND PREFERRED EMBODIMENTS

The broadest aspect of the invention is a method for preparing liposomes with high capture efficiency containing aqueous-soluble medicaments, which method comprises
lyophilizing a liposome-forming lipid dissolved in a suitable organic solvent to form a lyophilized lipid; and
hydrating the lyophilized lipid with an aqueous solution containing a suitable aqueous-soluble medicament.

A preferred class of the invention is the method wherein said aqueous-soluble medicament is an active protein or polypeptide, especially where such protein is interferon. A preferred subclass is that wherein said interferon is βIFN. A preferred species of the invention is the method wherein said βIFN is HuβIFN, especially rHuβ$IFN_{ser17}$.

Another aspect of the invention is an injectable, pharmaceutically acceptable suspension of liposomes encapsulating active interferon, wherein said liposomes remain near the injection site and release interferon over an extended period of time, e.g., 1 to 14 days. A preferred subgenus of the invention is the suspension wherein said suspension is formed by
lyophilizing a liposome-forming lipid dissolved in a suitable organic solvent to form a lyophilized lipid; and
hydrating the lyophilized lipid with an aqueous solution containing interferon. A preferred class is the method wherein said interferon is βIFN, especially HuβIFN. A preferred embodiment is the method wherein said HuβIFN is rHuβ$IFN_{ser17}$. Another preferred class is that wherein said lipid is a mixture of lipids. A preferred embodiment is that wherein said lipid mixture comprises diarachidoylphosphatidylcholine (DAPC) or distearoylphosphatidylcholine (DSPC) and dipalmitoylphosphatidylglycerol (DPPG), in a molar ratio of about 7:3. Another preferred class is that wherein said lipid is a compound of formula I:

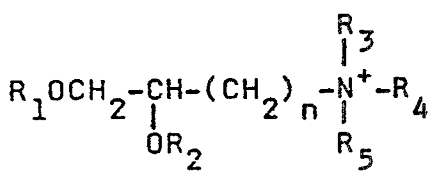

$X^-$ (I)

wherein $R_1$ and $R_2$ are independently alkyl or alkenyl of 6-22 carbons; $R_3$, $R_4$ and $R_5$ are each independently hydro, alkyl of 1-8 carbons, aryl or arylalkyl of 7-11 carbons; n is an integer from 1 to 8; and X is a pharmaceutically acceptable anion.

The preferred compounds of formula I are those wherein $R_1$ and $R_2$ are the same and are alkyl of 10-20 carbon atoms, or alkenyl of 10-20 carbon atoms; $R_3$, $R_4$ and $R_5$ are methyl or ethyl; n is 1-4 and X is a halide ion. Also preferred are those compounds wherein $R_1$ is alkyl or alkenyl of 14-22 carbon atoms and $R_2$ is alkyl or alkenyl of 6-14 carbon atoms.

Most preferred are the following compounds:
2,3-di(octadecyloxy)propyl-1-trimethylammonium chloride;
2,3-di(9-(Z)-octadecenyloxy)propyl-1-trimethylammonium chloride;
2,3-di(4-(Z)-decenyloxy)propyl-1-trimethylammonium chloride;
2,3-di(hexadecyloxy)propyl-1-trimethylammonium chloride;
2,3-di(decyloxy)propyl-1-trimethylammonium chloride;
2-hexadecyloxy-3-decyloxypropyl-1-trimethylammonium chloride;
2-hexadecyloxy-3-decyloxypropyl-1-dimethylammonium hydrochloride;
9,10-di(decyloxy)decyl-1-trimethylammonium chloride;
5,6-di(9,(Z)-octadecenyloxy)hexyl-1-trimethylammonium chloride; and
3,4-di(9-(Z)-octadecenyloxy)butyl-1-trimethylammonium chloride.

Another aspect of the invention is a method for effecting the sustained administration of interferon to a mammal in need thereof, which method comprises injecting an injectable, pharmaceutically acceptable

suspension of liposomes encapsulating active interferon, wherein said suspension is prepared by lyophilizing a liposome-forming lipid dissolved in a suitable organic solvent to form a lyophilized lipid; and hydrating the lyophilized lipid with an aqueous solution containing interferon. A preferred method is that wherein said interferon is βIFN, especially HuβIFN. A preferred embodiment is that wherein said HuβIFN is rHuβ$IFN_{ser17}$.

Another aspect of the invention is an easily prepared, pharmaceutically acceptable suspension of liposomes encapsulating active interferon suitable for topical application, wherein said liposomes remain near the application site and release interferon over a period of 1 to 14 days. A preferred class is the suspension wherein said interferon is βIFN, particularly HuβIFN, and especially rHuβ$IFN_{ser17}$. A preferred embodiment is that wherein said suspension is suitable for aerosol or "spray-on" application. Another preferred embodiment is that wherein said suspension is suitable for ophthamologic application, e.g., as eye drops. Another preferred embodiment is that wherein said suspension is suitable for administration as nasal drops or aerosol. Another preferred embodiment is that wherein said suspension is suitable for topical administration, e.g., for the treatment of cervical condyloma or genital herpes.

Another aspect of the invention is a method for effecting the sustained administration of interferon to a mammal in need thereof, which method comprises topically administering an easily prepared, pharmaceutically acceptable suspension of liposomes encapsulating active interferon suitable for topical application, wherein said suspension is prepared by
lyophilizing a liposome-forming lipid dissolved in a suitable organic solvent to form a lyophilized lipid; and hydrating the lyophilized lipid with an aqueous solution containing interferon. A preferred class is the suspension wherein said interferon is βIFN, especially HuβIFN. A preferred embodiment is the suspension wherein said HuβIFN is rHuβ$IFN_{ser17}$.

The following examples illustrate the practice of this invention, but are not intended to limit the scope of the invention in any way.

UTILITY AND ADMINISTRATION

Interferon is known to exhibit antiviral activity when administered to mammals. For example, the composition of the present invention exhibits excellent activity against rhinoviruses and coronaviruses, herpes simplex virus 1 and 2 and related viruses such as cytomegalovirus, Epstein-Barr virus and varicella zoster virus, as well as viral hepatitis such as hepatitis B.

The composition of the invention may be administered parenterally (usually by subcutaneous or intramuscular injection) or topically. "Topical" administration, as used herein, includes administration directly to a skin lesion, e.g., as a cream, sprayed-on or swabbed-on suspension, administration to the nasal mucosa as an aerosol, administration to the cervix, vaginal, and external genitalia, and administration anorectally in the form of suppositories or the like. The suspensions of the invention may also be administered for the treatment of lesions in the mouth, e.g., sublingually.

βIFN is administered parenterally in LMLV suspension at dose levels of 10 to $10^7$ U/kg, preferably $10^3$ to $10^5$ U/kg of body weight in man, administered once or twice a day for 1 to 7 days per week. For eye drops, the interferon is present at $10^3$-$10^9$ U/ml, preferably about $10^5$-$10^8$ U/ml.

For infections of the eye or other external tissues, e.g. mouth, skin, and genitalia, the compositions are preferably applied to the infected part of the patient's body topically as a cream, aerosol or suspension, preferably in an aerosol or cream. For aerosol administration, the suspension is preferably applied without using a propellant, e.g., by using a pump or squeeze-type sprayer.

Cream-like formulations may be prepared simply by using high concentrations of lipids. For example, a liposome formulation containing greater than about 130 mg/ml lipid will have a cream-like character. Alternatively, a more diluted liposome formulation may be thickened using gelling agents known to those skilled in the art. Cream formulations will contain from about $10^3$ U/ml to about $10^9$ U/ml, preferably about $10^6$ to $10^8$ U/ml.

Ophthalmic preparations are sterile products for either topical application to the eyes or instillation into the space (cul-de-sac) between the eyeball and the eyelids. An excipient is ophthalmologically acceptable if it is non-irritating to the eye and non-toxic at effective levels. The amount of active medicament will vary with the particular formulation and disease state but generally will be between 0.0001-10% wt/vol of active ingredient per individual application dose, preferably between 0.005-1% wt/vol.

Pharmaceutical ophthalmic compositions are typically provided as sterilized aqueous solutions (i.e. eyedrops) containing 0.0001% to 10% wt/vol.; most preferably 0.005% to 1% of the active ingredient, along with a suitable buffer, stabilizer, and preservative. The total concentration of solutes should be such that, if

possible, the resulting solution is isotonic with the lacrimal fluid (though this is not absolutely necessary) and has an equivalent pH (in the range of pH 6-8). Typical preservatives/sterilants are phenylmercuric acetate, thimerosal, chlorobutanol, and benzalkonium chloride. Typical buffer systems and salts are based on, for example, citrate, borate or phosphate.

The compositions of the invention may also be administered by other nonsystemic modes. Ophthalmic packs may be used to give prolonged contact of the solution with the eye. For example, a cotton pledget saturated with an ophthalmologically suitable suspension of a compound of this invention may be inserted into the superior or inferior fornix.

The following Preparations illustrate the production of several compounds of formula I.

PREPARATION 1

1,3:4,6-Di-O-benzylidine-D-mannitol

Concentrated sulfuric acid (40 ml) was added to a solution of D-mannitol (200 g) and benzaldehyde (240 ml) in dimethylformamide (600 ml). After stirring this solution at room temperature for 3 days, the mixture was poured into a stirred mixture of ice water (6 L), potassium carbonate (60 g) and petroleum ether (1 L). The resulting solid was collected by filtration, washed with petroleum ether and triturated with hot chloroform to give the title compound, m.p. - 190-191 °C.

PREPARATION 2

2,5-Di-O-benzyl-1,3:4,6-di-O-benzylidene-D-mannitol

Powdered potassium hydroxide (37 g) was added to 1,3:4,6-O-benzylidene-D-mannitol (10 g) dissolved in benzyl chloride (64 ml). The mixture was heated at 140 °C for 3 h, then cooled and diluted with water (200 ml). Extraction with chloroform, followed by washing with water and evaporation gave a solid, which was crystallized from petroleum ether to give the captioned compound, m.p. - 102-3 °C.

PREPARATION 3

2,5-Di-O-benzyl-D-mannitol

2,5-Di-O-benzyl-1,3:4,6-di-O-benzylidene-D-mannitol (10.9 g) dissolved in ethanol (150 ml) and water (22 ml) was treated with IM HCl (7 ml). After refluxing this mixture for 4.5 h, the reaction was cooled and quenched with barium carbonate, then evaporated to dryness. The solid residue was triturated with hot ethyl acetate, which was then evaporated to give the captioned compound, mp - 116-117 °C.

PREPARATION 4

1,2,10-Decanetriol

9-Decen-1-ol (25.0 g, 160 mM) was dissolved in a solution up of t-butanol (100 ml), acetone (90 ml) and water (10 ml). To this solution was added trimethylamine-N-oxide (26.6 g, 240 mM) and 2 ml of a solution of osmium tetroxide (500 mg) in t-butanol (25 ml). The resulting solution was stirred 20 h under nitrogen then 10% sodium bisulfite was added (50 ml). The mixture was concentrated, then taken up in trichloromethane and washed twice with water, dried with $Na_2SO_4$ and concentrated to give 1,2,10-decanetriol as an oil. This material was used without further purification in preparation of the acetonide.

PREPARATION 5

2,5-Dibenzyl-D-mannitol-3,4-acetonide

2,5-Dibenzyl-D-mannitol (48 g, 133 mM) dissolved in dry acetone (1000 ml) was treated with copper(II)-sulfate (10 g, 62.6 mM) and concentrated sulfuric acid (2 ml). After stirring at room temperature for 48 h, the mixture was quenched by the addition of solid sodium carbonate, followed by stirring for 3 hours. The reaction mixture was filtered and concentrated and the residue crystallized from hexane ethyl acetate to give 38.0 g of the title compound, m.p. 73-74° C.

PREPARATION 6

2,5-Dibenzyl-1,6-didecyl-D-mannitol-3,4-acetonide

A mixture of 2,5-dibenzyl-D-mannitol-3,4-acetonide (10.0 g, 25 mM), powdered KOH (23 g) and decyl bromide (40 ml) in xylene (300 ml) was heated at reflux for 4 h. The mixture was cooled, diluted with hexane (300 ml), decanted from excess salts and applied to a column of dry silica gel (1 Kg). Elution with hexane followed by a gradient of 0 to 50% ether in hexane gave the title compound as an oil.

PREPARATION 7

1,6-Didecyl-D-mannitol-3,4-acetonide

Dibenzyl compound of Preparation 6 (6.0 g, 8.8 mM) was dissolved in tetrahydrofuran/methanol (1:1, 100 ml). After bubbling nitrogen through for several minutes, 10% palladium on carbon (1 g) was added and the mixture was shaken at 70° C under 60 psi hydrogen for 48 h. The mixture was filtered and concentrated to give the title compound (4.3 g) as a white solid; m.p. 36-39° C.

PREPARATION 8

1,6-Didecyl-2,5-dihexadecyl-D-mannitol-3,4-acetonide

1,6-Didecyl-D-mannitol-3,4-acetonide (4.3 g, 8.57 mM) and bromohexadecane (7.84 g, 25.7 mM) were dissolved in xylene (40 ml) and KOH (5.0 g) was added. This mixture was stirred at reflux for 1.5 h. After cooling the mixture was decanted onto a column of dry silica gel (200 g), then eluted with hexane followed by 3% ether in hexane to give the title compound (7.3 g) as an oil.

PREPARATION 9

1,2,5,6-Tetraoleoyl-D-mannitol-3,4-acetonide

D-Mannitol-3,4-acetonide (5.0 g, 22.52 mM) was dissolved in dimethylformamide (200 ml, distilled from calcium hydride under reduced pressure). To this solution was added sodium hydride (1.08 g, 22.52 mM, 50% oil dispersion) and the mixture was heated to 50° C and stirred for 1 h (mechanical stirrer required). To the resulting mixture was added the toluenesulfonate of oleyl alcohol (9.5 g, 22.52 mM). The temperature was increased to 90° C and stirring was continued for 1 h.

The sequence of addition of NaH (same amount) and stirring 1 h, then addition of oleyl tosylate (same amount) and stirring 1 h, all at a constant 90° C, was repeated 4 more times (total of 5 times). The reaction mixture was allowed to cool to room temperature than poured slowly into a saturated solution of NaCl (500

ml). The resulting mixture was extracted with hexane (3 x 250 ml), dried over $K_2CO_3$, and concentrated. The crude product was chromatographed over silica gel (1000 g) eluting with a gradient of from 0 to 5% diethyl ether in hexane to give 13.93 g of the title compound as a viscous oil.

PREPARATION 10

1,2,5,6-Tetraoleoyl-D-mannitol

To a solution of 1,2,5,6-tetraoleoyl-D-mannitol-3,4-acetonide (24.0 g, 19.62 mM) in tetrahydrofuran (100 ml) was added $H_2O$:trifluoroacetic acid (1:9, 100 ml). This solution was stirred for 1 h at 50° C, then concentrated to an oil by rotary evaporation. Toluene (200 ml) was added and evaporated to azeotropically remove the residual acid. The crude material was dissolved in diethyl ether (100 ml) and a saturated solution of $NH_4OH$ in water (10 ml) was added. This mixture was stirred for 2 h and then the ether phase was washed twice with water, dried over $MgSO_4$, and concentrated. The crude product was suitable for further reaction; a small portion was purified by column chromatography over silica gel (10% ethyl acetate/hexane) to give an analytical sample of the desired diol as a viscous oil.

PREPARATION 11

2,3-Di(9-(Z)-octadecenyloxy)propyl-1-dimethylaminopropane

The crude dial 1,2,5,6-tetraoleoyl-D-mannitol described in the previous Preparation was dissolved in chloroform (500 ml) and lead tetraacetate (11.8 g, 26.0 mM) was added. This mixture was stirred for 2 h and then ethylene glycol (5 ml) was added followed quickly by water (100 ml). The water phase was drawn off and the organic phase was washed once with saturated NaCl solution, dried over $MgSO_4$, and concentrated to an oil to give the crude aldehyde which was used immediately in the next step.

To a solution of dimethylamine hydrochloride (35.5 g, 435 mM) in methanol (150 ml) was added anhydrous sodium acetate (24 g, 282 mM). The mixture was stirred for 1 h and then the resulting NaCl was filtered off and the clear methanol solution added to the crude aldehyde. Tetrahydrofuran (150 ml) was added followed by 3 angström molecular sieves (about 20 g). Sodium cyanoborohydride (1.5 g, 23.9 mM) was added and the mixture stirred at 50° C for three days. The crude reaction mixture was filtered through celite (washing with tetrahydrofuran) and the solution strongly acidified with IN HCl and stirred for 1/2 h. The solution was then made strongly basic with 10% NaOH and extracted with diethyl ether (3 x 200 ml). The crude product was purified by column chromatography over silica gel using a gradient of 0 to 10% methanol in chloroform to give the captioned dimethylamino product as a viscous oil.

The hydrochloride salt of the title compound was prepared by dissolving the foregoing product (100 mg) in ether (10 ml) and adding three drops of ethyl acetate saturated with HCl gas. The resulting solution was concentrated and placed under high vacuum for 24 h. The resulting product was a gummy solid.

PREPARATION 12

2,3-Di(9-Z-octadecenyloxy)propyl-1-trimethylammonium chloride

The dimethyl amino product 2,3-di(9-(Z)-octadecenyloxy)propyl-1-dimethylaminopropane (10 g) was placed in a Parr pressure reactor and cooled to -78° C. Methyl chloride (about 50 ml) was condensed into the reaction vessel, which was then sealed and heated to 70° C for 48 h. The reaction vessel was cooled and opened and the methyl chloride allowed to evaporate under a stream of nitrogen. The crude product was crystallized from acetonitrile to give the title compound as an off white solid, m.p. 65-72° C.

Proceeding in a similar manner, but substituting for 2,3-di(9-(Z)-octadieneyloxypropyl-1-dimethylaminopropane the appropriate precursor, the following compounds are made:
2,3-didecanyloxypropyl-1-trimethylammonium chloride; and
2,3-didocosanyloxypropyl-1-trimethylammonium chloride.

PREPARATION 13

2,3-Di(9-(Z)-octadecenyl)propanol

Crude 2,3-di(9-(Z)-octadecenyl)propanal (10.0 g, 16.9 mM) was dissolved in tetrahydrofuran/methanol (1:1, 200 ml) and cooled to 0 °C. Sodium borohydride (3.13 g, 85.0 mM) was added and the mixture was stirred overnight. The solution was acidified with IN HCl to pH<2, diluted with ether, washed with water, concentrated and column chromatographed (chloroform) to give the title compound as an oil.

PREPARATION 14

1,2-Di(9-(Z)-octadecenyloxy)-3-iodopropane

The alcohol 2,3-di(9-(Z)-octadecenyl)propan-1-ol (5.0 g, 8.36 mM) was dissolved in pyridine (50 ml) and p-toluenesulfonyl chloride (1.91 g, 10.0 mM) was added. The solution was stirred for 24 h, then poured into ice water, extracted with ether and washed with IN HCl until the aqueous layer remained acidic. The organic phase was dried over $MgSO_4$, and concentrated to give crude tosylate. The material was dissolved in methyl ethyl ketone (50 ml), NaI (1.5 g, 10.0 mM) was added and refluxed for 5 hours. The solvent was stripped and the residue was taken up in ether and washed with water. The organic layer was concentrated and chromatographed to give the title compound as an oil.

PREPARATION 15

2,3-Di(9-Z-octadecenyloxy)propyl-1-N-quinuclidinium chloride

The iodide of Preparation 6 (2.0 g, 2.82 mM) was dissolved in dichloromethane (1 ml) and quinuclidine (1.57 g, 14.1 mM) was added. The solution was sealed in a pressure reactor and heated to 100 °C for 48 h. The crude product was chromatographed over a small plug of silica gel (0 to 5% methanol in chloroform) and then ion exchanged over dowex 2-X8 (chloride form, eluting with methanol) to give the title compound as an oil.

PREPARATION 16

1,2,6-Hexanetriol-1,2-acetonide

1,2,6-Hexanetriol (31 g, 0.23 mM) was stirred with acetone (150 ml). To this mixture was added concentrated sulfuric acid (5 drops). The resulting solution was stirred for 2 h at room temperature. The reaction solution was diluted with diethyl ether, washed with saturated sodium bicarbonate solution, dried over $MgSO_4$, and concentrated to give the title compound (31 g) as a clear oil.

PREPARATION 17

1,2-Hexanediol-1,2-acetonide-6-allyl ether

The acetonide of Preparation 16 (30 g, 172 mM) was dissolved in dry dimethylformamide (500 ml). To this solution was added NaH (8.28 g, 172 mM, 50% oil dispersion) and the mixture was stirred for 1/2 h at room temperature, then warmed to 90 °C over 1/2 h. To this mixture was added allyl chloride (21 ml, 258 mH) and the stirring was continued for 1 h. After cooling, the mixture was poured into water and extracted with ether (2 x 100 ml). The combined ether extracts were washed with brine, dried over $MgSO_4$, and

concentrated. Chromatography over silica gel (10% ether in hexane) gave the title product as a clear oil; bp=70 ° C at 0.01 mmHg.

PREPARATION 18

1,2-Hexanediol-6-allyl ether

In ethanol (100 ml) was dissolved 1,2-hexanediol-1,2-acetonide-6-allyl ether (20 g, 93.9 mM) to which was added 20 ml of IN HCl. The solution was then heated to 50 ° C for 2 hours. The resulting solution was concentrated, then taken up in chloroform (100 ml) and washed with brine (2 x 10 ml), dried over $Na_2SO_4$, and concentrated to give the title compound as a clear oil.

PREPARATION 19

1,2-Di(9-(Z)-octadecenyloxy)-6-allyloxyhexane

The diol of Preparation 18 (3.45 g, 19.83 mM) was dissolved in dry dimethylformamide (60 ml). To this solution was added NaH (951 mg, 19.8 mM). The mixture was heated to 90 ° C and oleoyl tosylate (8.37 g, 19.8 mM) was added. Stirring was continued for 1 h at which time a second equivalent of NaH (951 mg, 19.8 mM) was added. After 15 minutes a second equivalent of oleyl tosylate (8.37 g, 198 mM) was added and stirring was continued for 1 h. The reaction mixture was poured into water and extracted with ether (2 x 100 ml). Column chromatography over silica gel (0 to 5% ether/hexane) gave 3.5 g of the title compound as a clear oil.

PREPARATION 20

1,2-Di(9-(Z)-octadecenyloxy)-hexan-6-ol

The triether of Preparation 19 (3.20 g, 4.74 mM) was dissolved in ethanol/tetrahydrofuran (1:1, 30 ml) and Wilkinsons catalyst (tris(triphenylphosphine)rhodium chloride, 200 mg) was added, followed by 0.IN HCl (1 ml). This mixture was refluxed for 3 h, then I N HCl (5 ml) was added and refluxed 4 h. The solution was cooled and concentrated. Diethyl ether was added and washed with brine, dried over $MgSO_4$, concentrated and chromatographed over silica gel (5 to 50% ether in hexane) to give 2.56 g of the title alcohol as an oil.

PREPARATION 21

6-Dimethylamino-1,2-di(9-(Z)-octadecenyloxy)hexane

The substituted hexan-6-ol from Preparation 20 (2.50 g, 3.94 mM) was dissolved in pyridine (20 ml) and p-toluenesulfonyl chloride (0.90 g, 4.73 mM) was added. This mixture was stirred overnight at room temperature then poured into ice water and stirred 1/2 h. The resulting mixture was extracted with ether, and the ether phase was washed with 0.IN HCl, dried over $MgSO_4$, and concentrated. This crude intermediate was immediately dissolved in dimethylamine and placed in a sealed tube at room temperature for 20 h. The tube was cooled to 0 ° C and opened. The dimethylamine was allowed to evaporate under a stream of nitrogen. Column chromatography of the crude product over silica gel (0 to 5% methanol in chloroform) gave the title product as a very thick oil. The hydrochloride was prepared as described in Preparation 3. This was also an oil.

EXAMPLE 1

Murine Gamma Interferon-Liposome Preparation

14 Micromoles (μM) of dioleoylphosphatidylcholine (DOPC) and 6 μM of dioleoylphosphatidylglycerol (DOPG) were dissolved in 2 ml of chloroform and then dried down under a stream of nitrogen. The resulting dried films were placed under vacuum for 1/2 hour, after which each film was then dissolved into 1 ml of cyclohexane, transferred to a 100 ml round-bottomed flask and frozen on dry ice. The flasks were then attached to a lyophilization apparatus and the cyclohexane removed. Murine gamma interferon, 0.2 ml (500,000 units/ml) was suspended in 0.2 M glycine buffer at pH 3.5. This solution was then added to the lyophilized lipids to form liposomes. The liposomes were then diluted to a convenient concentration with more glycine buffer as needed.

EXAMPLE 2

Human Beta Interferon-Liposome Formulation

Lyophilized multilamellar vesicles (LMLV) containing recombinant human β-interferon with the amino acid residue at position 17 replaced by serine ($rHu\beta IFN_{ser17}$) were prepared using DAPC:DPPG as the lipid vehicle. The $rHu\beta IFN_{ser17}$ was obtained from Cetus Corporation, Emeryville, California and Triton Biosciences Inc., Alameda, California. Other sources of βIFN are also suitable, including natural and recombinant interferons. LMLVs were prepared by first drying the lipids (DAPC:DPPG, 7:3 molar ratio) from a chloroform solution to a film under a stream of nitrogen, followed by vacuum dessication for 30 minutes. Twenty μmoles total dried lipids were then dissolved in 1 ml cyclohexane, frozen, and the solvent removed by lyophilization. The lyophilized lipids were hydrated with 1 ml of an aqueous solution of βIFN containing $10^7$ U per milliliter. The final concentration of lipid was 20 mM. The formulations can be prepared with $10^3$-$10^9$ U IFN/ml, and the lipid concentration may range from 5-40 mM. The aqueous solution of βIFN used in the hydration procedure as described above can contain suitable, pharmaceutically acceptable stabilizers (such as human serum albumin and dextrose) and buffers.

EXAMPLE 3

Incorporation of βIFN Into Liposomes

The degree of βIFN incorporation in the liposomes was quantitated by spiking the βIFN with $^{125}$I-labeled βIFN. The LMLV-βIFN preparation was pelleted by centrifugation at 100,000 x g for 30 min., and the radioactivity in the supernatant and pellet was quantitated by gamma counting. With free, non-liposomal βIFN, all the counts remain in the supernatant. With the LMLV-βIFN preparation however, all of the counts pelleted with LMLV fraction thus demonstrating that the βIFN was incorporated in the liposomal preparation. The βIFN in the liposome preparation retained full antiviral activity, as determined by standard βIFN assays well known in the art.

EXAMPLE 4

Controlled Release of βIFN

The controlled release of βIFN from the LMLV-βIFN preparation was demonstrated after subcutaneous or intramuscular injection of the LMLV-βIFN (which contained a spike of $^{125}$I-βIFN) into the abdominal side (for sc) or the hind thigh muscle (for im) of a mouse. Fifteen μl of LMLV-βIFN was injected per mouse in two different sites. Two mice per time point were sacrificed at various times after injection and radioactivity remaining at each of the injections sites was determined by gamma counting. Another group of mice was similarly injected with the $^{125}$I-βIFN solution without liposome formulation, and the retention at the local injection site was determined as a function of time as described for the LMLV-βIFN preparations.

As shown in Table 1, the free βIFN was largely gone from the initial injection site after one day (7%

remaining after one day). However, the LMLV-$\beta$IFN was retained at the initial injection site as a depot which was gradually released over the following days. Thus 79% remained after one day, and even after 6 and 9 days, 18 and 6% respectively still remained. Thus, the $\beta$IFN from the LMLV-$\beta$IFN preparation was released in a controlled fashion over a 1-2 week period resulting in a sustained dosage of $\beta$IFN to the animal over this time period. This is in contrast to free $\beta$IFN, which was all largely released from the injection site after one day.

TABLE 1

| Composition | Day | $^{125}$I-$\beta$IFN Retained at Injection Site (a) cpm (b) | % of Initial |
|---|---|---|---|
| Free $\beta$IFN (without LMLV) | 0 | 13660 ± 1540 | ≡ 100.0 |
| | 1 | 1010 ± 170 | 7.4 |
| | 2 | 420 ± 80 | 3.1 |
| | 3 | 330 ± 30 | 2.4 |
| | 6 | 210 ± 30 | 1.6 |
| | 9 | 250 ± 180 | 1.9 |
| LMLV-$\beta$IFN (c) | 0 | 12930 ± 1710 | ≡ 100 |
| | 1 | 10180 ± 850 | 79 |
| | 2 | 7060 ± 650 | 55 |
| | 3 | 4860 ± 1480 | 38 |
| | 6 | 2320 ± 1320 | 18 |
| | 9 | 770 ± 90 | 6 |

(a) Interferon preparations were injected intramuscularly into the hind thigh of a mouse.

(b) (mean ± S.D.) Four samples per determination

(c) Composition of the LMLV (lyophilized multilamellar vesicles) was 7:3 DAPC:DPPG

EXAMPLE 5

Improvement of Encapsulation Efficiency

A series of vials are prepared, each containing 13.3 mg of lyophilized lipid (9.5 mg DOPC, 3.3 mg DOPG, 3.3 mg cholesterol) or dried lipid film (9.5 mg DOPC, 3.3 mg DOPG, 3.3 mg cholesterol). The lipids are then suspended in increasing volumes of buffer solutions containing either glucose-6-phosphate dehydrogenase (G6PDH) or radioiodinated interferon ($^{125}$I-rHu$\beta$IFN$_{ser17}$) to form MLVs. The final lipid concentration obtainable with lyophilized lipids is > 120 mg/ml, whereas the final lipid concentration obtainable with dried film lipids is < 50 mg/ml. MLV Encapsulation efficiency increases with increasing lipid concentration, so that the lyophilized lipids afford the highest encapsulation efficiency. However, it is also found that the lyophilized liposomes afford a higher encapsulation efficiency than dried film liposomes at the same lipid concentration.

## EXAMPLE 6

### Particle Size Distribution

Liposomes are prepared as in Example 5 above. A comparison of the particle size distribution as determined by laser light scattering or field flow fractionation (see Science, 215, 296-298 (1982)) indicates that the lyophilization process of the invention affords a distribution of MLVs which are larger than the MLVs prepared from dried lipids.

## EXAMPLE 7

### Formulations

The following examples illustrate the preparation of representative pharmaceutical formulations containing the composition of the invention.

**Intraocular Formulation**

| | | |
|---|---|---|
| rHuβIFN | | $2.0 \times 10^8$ U |
| DAPC:DPPG (7:3) | | 2.0 mmoles |
| Benzalkonium Chloride | | 0.01 g |
| EDTA | | 0.10 g |
| Sodium Phosphate Buffer | qs | pH 7.4 |
| 2% Boric acid solution | qs | 100 ml |

The lipids are lyophilized as described above, and hydrated with a solution containing the remaining components. The suspension is then filtered through a 0.2 micron membrane filter and packaged under sterile conditions.

**IV, IP, SC, IL and IM Formulation**

| | |
|---|---|
| rHuβIFN | $2 \times 10.0^8$ U |
| Human serum albumin | 1.25 g |
| Dextrose | 1.25 g |
| DAPC:DPPG (7:3) | 2.0 mmoles |
| 0.9% Saline solution qs | 100 ml |

The lipids are lyophilized as described above, and are hydrated with the rHuβIFN solution to form the liposomal composition of the invention. The suspension is filtered through a 0.2 micron membrane filter and packaged under sterile conditions.

**Topical Cream Formulation**

| | |
|---|---|
| rHuβIFN | $2 \times 10.0^8$ U |
| Human serum albumin | 1.25 g |
| Dextrose | 1.25 g |
| DAPC:DPPG (7:3) | 20.0 mmoles |
| 0.9% Saline solution qs | 100 ml |

The lipids are lyophilized as described above, and are hydrated with the rHuβIFN solution, titurating if necessary, to form the liposomal composition of the invention. The suspension is filtered through a 0.2 micron membrane filter and packaged under sterile conditions.

## Claims

1. A method for preparing liposomes containing aqueous-soluble medicaments, which method comprises lyophilizing a liposome-forming lipid dissolved in a suitable organic solvent to form a lyophilized lipid; and
hydrating the lyophilized lipid with an aqueous solution containing a suitable aqueous-soluble medicament.

2. The method of Claim 1 wherein said aqueous-soluble medicament is an active interferon.

3. The method of Claim 2 wherein said interferon is βIFN, αIFN or γIFN.

4. The method of Claim 3 wherein said βIFN is HuβIFN, preferably $rHu\beta IFN_{ser17}$.

5. An injectable, pharmaceutically acceptable suspension of liposomes encapsulating active interferon prepared by a method of any one of claims 1 to 4, wherein said liposomes remain near the injection site and release interferon over a period of 1 to 14 days.

6. Pharmaceutically acceptable suspension of liposomes encapsulating active interferon suitable for topical application, wherein
said liposomes have been prepared by a method of any one of claims 1 to 4 and remain near the application site and release interferon over a period of 1 to 14 days.

7. The suspension of Claim 5 or Claim 6 wherein said suspension is formed by a method of Claim 2.

8. The suspension of claim 5 or Claim 6 wherein said interferon is βIFN, αIFN or γIFN, preferably HuβIFN, most preferably $rHu\beta IFN_{ser17}$.

9. The suspension of any one of Claims 5 to 8 wherein said lipid is selected from phosphatidylcholine derivatives and phosphatidylglycerol derivatives, preferably a lipid mixture comprising distearoylphosphatidylcholine or diarachidoylphosphatidylcholine and dipalmitoylphosphotidylglycerol, most preferably a mixture comprising distearoylphosphatidylcholine or diarachidoylphosphatidylcholine and dipalmitoylphosphotidylglycerol in a molar ratio of about 7:3.

10. A pharmaceutically acceptable suspension of liposomes encapsulating active interferon prepared by a method of any one of Claims 2, 3 and 4, for use in effecting the sustained topical administration of interferon to a mammal in need thereof.

11. The suspension of Claim 10 wherein said topical application is by nasal drops or aerosol.

12. The suspension of Claim 10 wherein said topical application is by suppository.

13. The suspension of any one of Claims 10, 11 and 12 wherein said lipid is selected from phosphatidylcholine derivatives and phosphatidylglycerol derivatives, preferably a lipid mixture comprising distearoylphosphatidylcholine or diarachidoylphosphatidylcholine and dipalmitoylphosphatidylglycerol, most preferably a lipid mixture comprising distearoylphosphatidylcholine or diarachidoylphosphatidylcholine and dipalmitoylphosphatidylglycerol in a molar ratio of about 7:3.

## Revendications

1. Procédé de préparation de liposomes contenant des médicaments hydrosolubles, qui consiste
à lyophiliser un lipide, apte à former les liposomes, dissous dans un solvant organique convenable pour former un lipide lyophilisé ; et
à hydrater le lipide lyophilisé avec une solution aqueuse contenant un médicament hydrosoluble convenable.

2. Procédé suivant la revendication 1, dans lequel le médicament hydrosoluble est un interféron actif.

3. Procédé suivant la revendication 2, dans lequel l'interféron est le $\beta$IFN, l'$\alpha$IFN ou le $\gamma$IFN.

4. Procédé suivant la revendication 3, dans lequel le $\beta$IFN est le Hu$\beta$IFN, de préférence le rHu$\beta IFN_{ser17}$.

5. Suspension injectable, pharmaceutiquement acceptable, de liposomes renfermant à l'état encapsulé un interféron actif, préparés par un procédé suivant l'une quelconque des revendications 1 à 4, dans laquelle lesdits liposomes restent à proximité du site d'injection et libèrent l'interféron en un temps de 1 à 14 jours.

6. Suspension, pharmaceutiquement acceptable, de liposomes renfermant à l'état encapsulé un interféron actif, convenant pour une application topique, dans laquelle lesdits liposomes ont été préparés par un procédé suivant l'une quelconque des revendications 1 à 4, et restent à proximité du site d'application et libèrent l'interféron en un temps de 1 à 14 jours.

7. Suspension suivant la revendication 5 ou la revendication 6, qui est formée par un procédé suivant la revendication 2.

8. Suspension suivant la revendication 5 ou la revendication 6, dans laquelle l'interféron est le $\beta$IFN, l'$\alpha$IFN ou le $\gamma$IFN, avantageusement le Hu$\beta$IFN, de préférencele rHu$\beta IFN_{ser17}$.

9. Suspension suivant l'une quelconque des revendications 5 à 8, dans laquelle le lipide est choisi entre des dérivés de phosphatidylcholine et des dérivés de phosphatidylglycérol, et est avantageusement un mélange de lipides comprenant de la distéaroylphosphatidylcholine ou de la diarachidoylphosphatidylcholine et du dipalmitoylphosphatidylglycérol, de préférence un mélange comprenant de la distéaroylphosphatidylcholine ou de la diarachidoylphosphatidylcholine et du dipalmitoylphosphatidylglycérol en un rapport molaire d'environ 7:3.

10. Suspension, pharmaceutiquement acceptable, de liposomes renfermant à l'état encapsulé un interféron actif, préparés par un procédé suivant l'une quelconque des revendications 2, 3 et 4, destinée à l'utilisation dans l'administration topique prolongée d'interféron à un mammifère nécessitant une telle administration.

11. Suspension suivant la revendication 10, dans laquelle l'application topique est effectuée au moyen de gouttes nasales ou d'un aérosol.

12. Suspension suivant la revendication 10, dans laquelle l'application topique est effectuée au moyen d'un suppositoire.

13. Suspension suivant l'une quelconque des revendications 10, 11 et 12, dans laquelle le lipide est choisi entre des dérivés de phosphatidylcholine et des dérivés de phosphatidylglycérol, et est avantageusement un mélange de lipides comprenant de la distéaroylphosphatidylcholine ou de la diarachidoylphosphatidylcholine et du dipalmitoylphosphatidylglycérol, de préférence un mélange de lipides comprenant de la distéaroylphosphatidylcholine ou de la diarachidoylphosphatidylcholine et du dipalmitoylphosphatidylglycérol en un rapport molaire d'environ 7:3.

## Ansprüche

1. Verfahren zur Herstellung von Liposomen, die wasserlösliche Medikamente enthalten, umfassend
das Lyophilisieren eines Liposomen-bildenden Lipids, das in einem geeigneten organischen Lösungsmittel gelöst ist, um ein lyophilisiertes Lipid zu bilden; und
das Hydratisieren des lyophilisierten Lipids mit einer wäßrigen Lösung, die ein geeignetes wasserlösliches Medikament enthält,

2. Verfahren nach Anspruch 1, worin das wasserlösliche Medikament ein aktives Interferon ist.

3. Verfahren nach Anspruch 2, worin das Interferon $\beta$IFN, $\alpha$IFN or $\gamma$IFN ist.

4. Verfahren nach Anspruch 3, worin das $\beta$IFN Hu$\beta$IFN, vorzugsweise rHu$\beta IFN_{ser17}$ ist.

5. Injizierbare, pharmazeutisch annehmbare, nach einem Verfahren von irgendeinem der Ansprüche 1 bis 4 hergestellte Suspension von Liposomen, die aktives Interferon einkapseln, worin die Liposomen in der Nähe der Injektionsstelle verbleiben und Interferon über einem Zeitraum von 1 bis 14 Tagen freisetzen.

6. Pharmazeutisch annehmbare, zur topischen Applikation geeignete Suspension von Liposomen, die aktives Interferon einkapseln, worin die Liposomen nach einem Verfahren von irgendeinem der Ansprüche 1 bis 4 hergestellt worden sind und in der Nähe der Applikationsstelle verbleiben und Interferon über einen Zeitraum von 1 bis 14 Tagen freisetzen.

7. Suspension nach Anspruch 5 oder Anspruch 6, worin die Suspension nach dem Verfahren von Anspruch 2 gebildet wird.

8. Suspension nach Anspruch 5 oder Anspruch 6, worin das Interferon $\beta$IFN, $\alpha$IFN oder $\gamma$IFN, bevorzugt Hu$\beta$IFN, am meisten bevorzugt rHu$\beta IFN_{ser17}$, ist.

9. Suspension nach irgendeinem der Ansprüche 5 bis 8, worin das Lipid ausgewählt ist aus phosphatidylcholin-Derivaten und Phosphatidylglycerin-Derivaten, vorzugsweise einer Lipidmischung, die Distearoylphosphatidylcholin oder Diarachidoylphosphatidylcholin und Dipalmitoylphosphatidylglycerin umfaßt, am meisten bevorzugt einer Mischung, die Distearoylphosphatidylcholin oder Diarachidoylphosphatidylcholin und Dipalmitoylphosphatidylglycerin in einem Molverhältnis von ungefähr 7:3 umfaßt.

10. Pharmazeutisch annehmbare, nach einem Verfahren von irgendeinem der Ansprüche 2, 3 und 4 hergestellte Suspension von Liposomen, die aktives Interferon einkapseln, zur Verwendung beim Bewirken der retardierten topischen Verabreichung von Interferon an einen Säuger, der dessen bedarf.

11. Suspension nach Anspruch 10, worin die topische Verabreichung durch Nasentropfen oder ein Aerosol geschieht.

12. Suspension nach Anspruch 10, worin die topische Verabreichung durch Suppositorien geschieht.

13. Suspension nach irgendeinem der Ansprüche 10, 11 und 12, worin das Lipid ausgewählt ist aus Phosphatidylcholin-Derivaten und Phosphatidylglycerin-Derivaten, vorzugsweise einer Lipidmischung, die Distearoylphosphatidylcholin oder Diarachidoylphosphatidylcholin und Dipalmitoylphosphatidylglycerin umfaßt, am meisten bevorzugt einer Lipidmischung, die Distearoylphosphatidylcholin oder Diarachidoylphosphatidylcholin und Dipalmitoylphosphatidylglycerin in einem Molverhältnis von ungefähr 7:3 umfaßt.